# EUROPEAN PATENT APPLICATION

(11) **EP 4 825 428 A1**
(43) Date of publication of application: **21.10.2026**
(21) Application number: 24903949.6
(22) Date of filing: 24.10.2024
(51) Int. Cl.: A61M 25/06

(54) **PIVOT-TYPE REUSE-PREVENTION CATHETER**

(30) Priority: 14.12.2023 KR 20230182005
(71) Applicant: MEDIFIRST CO., LTD., Chungcheongnam-do 31223 (KR)
(72) Inventor: AN, Chi Jeong, Cheonan-si Chungcheongnam-do 31223 (KR); KO, Kwon Cheul, Sejong 30005 (KR)
(74) Representative: Plougmann Vingtoft a/s
(86) International application number: PCT/KR2024/016271
(87) International publication number: WO 2025/127382

(57) **Abstract**

The present invention provides a reuse-prevention catheter comprising: a catheter hub having an outer needle provided therein; an inner needle inserted into the outer needle by passing through the catheter hub; a cannula plug having the inner needle provided therein, and which separates from the catheter hub while the inner needle is withdrawn from the outer needle; a slide case fastened to the cannula plug, accommodated inside the cannula plug, and discharged from the cannula plug so as to cover the inner needle; a block socket fitted to a rear end of the catheter hub, having a needle hole that communicates with the catheter hub, and coupled to a front end of the slide case; and a locking pivot accommodated inside the block socket, having a latching part formed on one side thereof to be engaged with the rear end of the catheter hub, and having a blocking part that extends from the latching part and is bent inwardly toward the block socket, wherein when the cannula plug separates from the catheter hub, the locking pivot is pivoted such that the blocking part blocks the needle hole.

## Description

### TECHNICAL FIELD

The present invention relates to a reuse-prevention catheter that is structurally incapable of being reused after use of the catheter and is capable of being safely discarded.

### BACKGROUND ART

Catheters are efficient tools for injecting drugs to subjects and are widely used in hospitals. The catheter is a configuration for leaving a hub provided with an outer needle made of a tube material at a front end thereof with respect to subjects who require frequent drug infusion such as patients. Thus, when a liquid medicine is injected through injection, it may provide convenience by eliminating the need to newly pierce a skin with a needle. In order to insert the catheter into the body of the subject, a needle of a cannula is inserted into the outer needle, and then, the catheter is inserted into the body of the subject together with the outer needle. Thereafter, the cannula is retracted to leave only the catheter hub, and the inner needle is removed.

In modern medicine, the reuse of invasive instruments is treated as a "prohibited matter" that must never be done. In particular, the cannula needle, which is directly related to a risk of infection, has to be used only once, regardless of a site of insertion. Since the needle is made very finely and is already deformed at a time point of single-use, there is a risk of causing excessive damage to tissues such as blood vessels when reused. Moreover, since the needle enters and exits the human body, there is a high possibility that the needle serves as a medium for infection.

Although the reuse of invasive disposable medical devices such as catheters is prohibited, individuals who are not professional medical personnel may reuse the used needles by heating the needles with fire due to lax hygiene knowledge and cost considerations. Thus, the invasive medical devices such as catheters need to be implemented in such a way that reusability is not possible structurally.

Korean Registered Patent No. 10-1598469, which is a prior registered patent of the present applicant, pertains to a catheter. The catheter has a non-reversible operation mechanism in which a gear, a rack, and teeth formed on a hub are used to fix or separate the hub from a movable bar. Then, after the needle is retreated to allow the movable bar and the hub to be separated from each other, the rack blocks a path for the needle to extend outward, thereby allowing the reuse of the needle to be impossible.

However, in the configuration of the pivot of the conventional reuse-prevention safety catheter, components such as the rack and the gear had to be accommodated to provide a pivot housing having a predetermined size. In addition, a blocking means that prevents the reuse was constituted by two pieces such as the rack and the gear to require a time and cost for assembly during manufacturing. It is obvious that the manufacturing cost of the catheter is directly related to its commercial viability due to its nature. However, it is not desirable to require a large number of injection-molded products and complex manufacturing processes for single-use implementation.

Thus, the present applicant simplified the structure through a design modification in foregoing Korean Registration Patent No. 10-1598469. In particular, while significantly reducing s volume of the pivot that prevents the reuse, the present applicant invented a reuse-prevention catheter that is easy to assemble and minimizes the number of components.

### DISCLOSURE OF THE INVENTION

### TECHNICAL PROBLEM

An object of the present invention is to provide a reuse-prevention catheter that minimizes a volume and number of components in a configuration required for a reuse-prevention configuration, which prevents reinsertion of a needle to facilitate assembly and enable production at low manufacturing costs.

### TECHNICAL SOLUTION

A reuse-prevention catheter according to the present invention includes: a catheter hub provided with an outer needle; an inner needle inserted into the outer needle by passing through the catheter hub; a cannula plug provided with the inner needle and separated from the catheter hub while the inner needle is withdrawn from the outer needle; a slide case coupled to the cannula plug, accommodated into the cannula plug, and discharged from the cannula plug to cover the inner needle; a block socket fitted into a rear end of the catheter hub, having a needle hole that communicates with the catheter hub, and coupled to a front end of the slide case; and a locking pivot accommodated inside the block socket, provided with a latching part engaged with a rear end of the catheter hub at one side thereof, and provided with a blocking part that extends from the latching part and is bent inward toward the block socket, wherein, when the cannula plug is separated from the catheter hub, the locking pivot is pivoted so that the blocking part blocks the needle hole.

According to an embodiment, the slide case may have a multistage structure including: a first slide case which is accommodated inside the cannula plug and is provided with a latching part configured to prevent the first slide case from being separated when being discharged from the cannula plug; and a second slide case which is accommodated inside the first slide case and is provided with a latching part configured to prevent the second slide case from being separated when being discharged from the first slide case.

According to an embodiment, the block socket may be divided into a first inner space, in which a front end on which a latching part of the locking pivot is disposed is accommodated, and a second inner space, in which a rear end on which a blocking part of the locking pivot is partially disposed and through which the needle hole communicates.

According to an embodiment, the locking pivot may be configured so that: a protrusion protruding from the latching part is disposed to be engaged with a rear end of the catheter hub in the first space of the block socket; the latching part rotates outward by external force, through which the cannula plug is separated from the catheter hub, to separate the catheter hub and the block socket from each other; and as the latching part rotates outward, the blocking part rotates inward in the second space to block the needle hole.

According to an embodiment, the reuse-prevention catheter may further include a socket cover configured to shield an upper portion of the block socket, wherein the socket cover may be provided with an anti-slip protrusion on a top surface thereof.

According to an embodiment, the block socket may be configured so that: a hub coupling part having the needle hole is provided on a front end of a socket housing that defines the second inner space; and a pivot housing that allows the first inner space to communicate in a ' 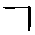 ' shape is provided on an upper portion of the socket housing.

According to an embodiment, the block socket may have a coupling hole defined in a sidewall of the pivot housing so that the locking pivot is rotatably coupled, and the locking pivot may be provided with a hinge part inserted into the coupling hole on a front end thereof so that the latching part provides a rotatable fixed end, and the blocking part provides a free end.

According to an embodiment, the locking pivot may have: a front end defined together with the latching part by including a hinge part coupled to the block socket; an extension part configured to transmit rotation force of the latching part; and a rear end defined by the blocking part bent from the extension part in an inward direction of the block socket.

According to an embodiment, in the locking pivot, the blocking part may be bent in the inner direction of the block socket so that an angle between the extension part and the blocking part is in range of an obtuse angle comprising an angle of 90°.

### ADVANTAGEOUS EFFECTS

According to the present invention, during the process of separating the cannula plug to leave the hub provided with the outer needle made of the tube material at the front end in the catheter, the locking pivot that is caught on the catheter hub may be pivoted to block the inside of the block socket. Since the single component of the locking pivot is sufficient as a means that prevents the reinsertion of the inner needle, the number of components may be minimized, and particularly, the volume of the block socket may be reduced.

According to the present invention, once the locking pivot is separated from the catheter hub, the latching part may be hidden inside the first inner space due to the pivoting of the locking pivot, and it may be impossible to restore the locking pivot that is pivoted within the block socket, and thus, the needle hole of the block socket may be shielded. Therefore, the safety catheter, which is capable of being manufactured with the minimized number of components to reduce the manufacturing costs and facilitate the assembly while preventing the reuse, may be provided.

### BRIEF DESCRIPTION OF THE DRAWINGS

FIG. 1 is a perspective view of a reuse-prevention catheter according to an embodiment of the present invention.
FIG. 2 is a view illustrating an operation state of the reuse-prevention catheter according to an embodiment of the present invention. FIG. 2(a) is a view illustrating a state when a cap is separated to pierce the catheter, and FIG. 2(b) is a view illustrating a state when a cannula plug is withdrawn after the piercing.
FIG. 3 is a side cross-sectional view of the reuse-prevention catheter according to an embodiment.
FIG. 4 is a view illustrating a block socket, a locking pivot, and a socket cover according to an embodiment of the present invention.
FIG. 5 is a view illustrating a state in which the cannula plug of the reuse-prevention catheter is completely withdrawn to allow the locking pivot to block a reinsertion path of an inner needle according to an embodiment of the present invention.
FIG. 6 is a view of the socket cover according to an embodiment of the present invention.
FIG. 7 is a view illustrating a block socket and a locking pivot according to another embodiment of the present invention. FIG. 7(a) is a transparent perspective view illustrating a block socket and a locking pivot according to another embodiment, FIG. 7(b) is a front view illustrating the block socket and the locking pivot according to another embodiment, and FIG. 7(c) is side view illustrating the block socket and the locking pivot according to another embodiment.

### MODE FOR CARRYING OUT THE INVENTION

The various embodiments described in this document are provided for the purpose of clearly illustrating the present invention and technical spirit of the present invention, and are not intended to limit the present invention to specific embodiments. The present invention and the technical concept disclosed herein include various modifications, equivalents, alternatives, and embodiments that are selectively combined from all or a portion of each embodiment described herein. In addition, the scope of the present invention and the technical concept disclosed herein are not limited to the embodiments presented below or the specific descriptions thereof.

Unless defined otherwise, terms used herein may have meanings commonly understood by those of ordinary skill in the art to which the present invention and disclosure belong, including technical and scientific terms.

The expressions "comprises", "may comprise", "is provided with", "may be provided with", "has", "may have", etc., used herein, mean that a feature, operation, or component is present as an object, and do not exclude the presence of other additional features. That is, such expressions should be understood as open-ended terms that imply the possibility of including other embodiments.

The singular form used in this document may include meanings of the plural forms unless otherwise indicated by context, and the same is applied to the singular form set forth in the claims.

The expression "A, B, and C," "A, B, or C," "A, B, and/or C," "at least one of A, B, and/or C," "at least one of A, B, and/or C," "at least one selected from A, B, and C," "at least one selected from A, B, and/or C," etc., as used in this document, may mean each of the listed items or all possible combinations of the listed items. For example, "at least one selected from A and B" may refer to (1) A, (2) at least one of A, (3) B, (4) at least one of B, (5) at least one of A and at least one of B, (6) at least one of A and B, (7) at least one of B and A, and (8) both A and B.

The expression "on the basis of" used in this document is used to describe one or more factors that affect decision, action of determination, or an operation described in the phrase or sentence in which this expression is included, and this expression does not exclude additional factors that affect the decision, the action of the determination, or the operation.

As used in this document, the expression that one element (e.g., a first element) is "connected to" or "coupled to" another element (e.g., a second element) may mean that the first element is directly connected or coupled to the second element, as well as being connected or coupled to the second element by using an intervening element (e.g., a third element) as a medium.

As used in this document, the expression "configured to" may have meanings such as "set to", "having the ability to", "modified to", "created to", or "capable of", depending on the context, and is distinguished from the meaning of "consist of".

Hereinafter, various embodiments of the present disclosure will be described with reference to the accompanying drawings. In the accompanying drawings and descriptions thereof, identical or substantially equivalent elements may be assigned the same reference numerals. In addition, in the description of various embodiments below, duplicated descriptions of identical or corresponding elements may be omitted. However, this does not imply that such elements are not included in the embodiment.

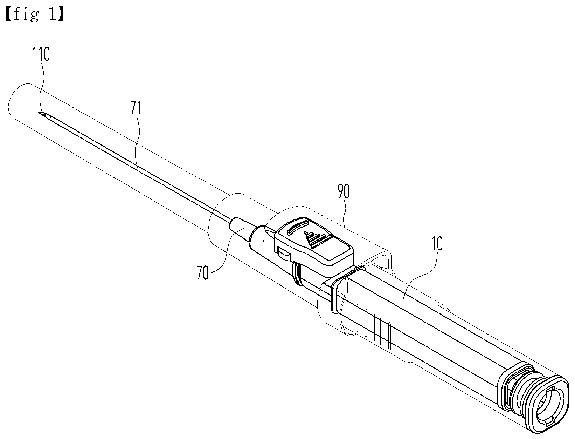

FIG. 1 is a perspective view of a reuse-prevention catheter according to an embodiment of the present invention.

Referring to FIG. 1, a reuse-prevention catheter may include a cap 90, a catheter hub 70, a cannula plug 10, an outer needle 71, and an inner needle 110. In this embodiment, the reuse-prevention catheter may further include a slide case 30, a block socket 40, a locking pivot 50, and a socket cover 44 and further included components will be described later through the drawings.

The cap 90 is coupled to the catheter hub 70 before the inner needle 110 is inserted into a body of a subject so as not to expose an end of the inner needle 110. In order to use the reuse-prevention catheter according to this embodiment, when the inner needle 110 is to be inserted into the body of the subject, the cap 90 is separated and removed.

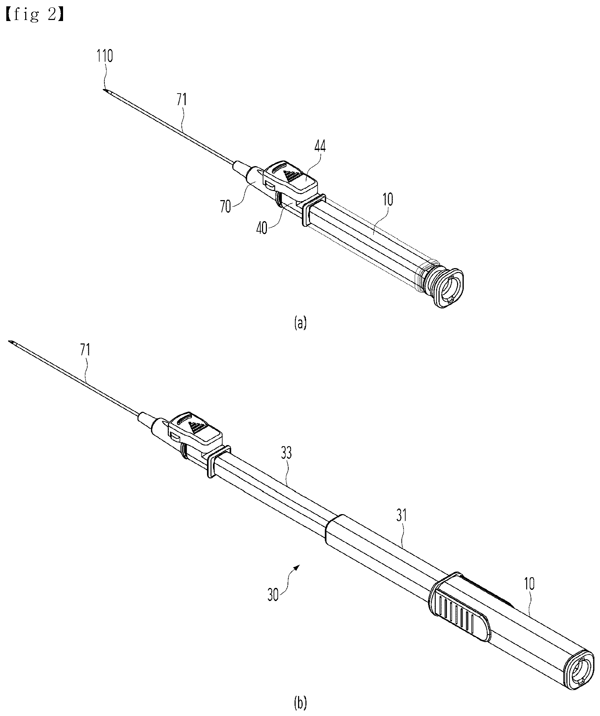

FIG. 2 is a view illustrating an operation state of the reuse-prevention catheter according to an embodiment of the present invention. FIG. 2(a) is a view illustrating a state when the cap 90 is separated to pierce the catheter, and FIG. 2(b) is a view illustrating a state when a cannula plug 10 is withdrawn after the piercing.

Referring to FIG. 2, after the inner needle 110 is pierced, and the outer needle 71 is intruded, the slide case 30 accommodated in the cannula plug 10 is withdrawn during the process of removing the inner needle 110. After the slide case 30 is sufficiently withdrawn, the catheter hub 70 is separated from the cannula plug 10, and the slide case 30 covers the periphery of the inner needle 110 that is withdrawn from the catheter hub 70. Once a single-use of the reuse-prevention catheter is completed, and the slide case 30 is completely withdrawn, a non-reversible mechanism has to be implemented to prevent the slide case 30 from being reinserted into the cannula plug 10. It is noteworthy that the slide case 30 according to this embodiment does not have a separate coupling structure for locking. However, even in the absence of the coupling structure, the slide case 30 is prevented from being reinserted in a direction of the cannula plug 10 since the locking pivot 50 blocks the end of the inner needle 110 within the block socket 40 as described below. Hereinafter, after describing the configuration of the reuse-prevention catheter according to the present embodiment, the aforementioned mechanism will be described in detail.

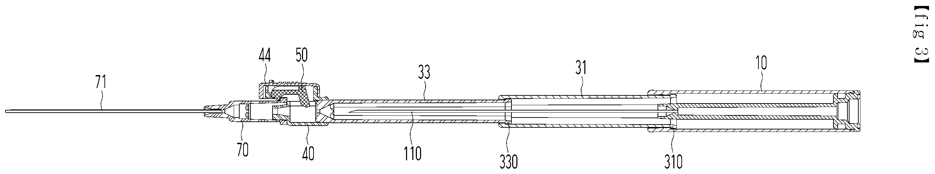

FIG. 3 is a side cross-sectional view of the reuse-prevention catheter according to an embodiment. FIG. 3 illustrates a state in which the slide case 30 is sufficiently withdrawn, i.e., a state just before the catheter hub 70 is separated.

The catheter hub 70 is provided with an outer needle 71 and is configured to leave the outer needle 71 made of a tube material after pierced as a guide for the inner needle 110 with respect to the subject who require frequent drug infusion. In this embodiment, a coupling means may be disposed on an outer circumference of a rear end of the catheter hub 70. The coupling means of the catheter hub 70 may be a stepped portion protruding outward. In general, since the protruding structure is provided on the outer circumference of the rear end of the catheter hub 70, into which a cannula is fitted, the block socket 40 that is fitted into the catheter hub 70 may also be applied to a typical catheter hub that is commonly available in the market. If the coupling means is not provided at all at the rear end of the catheter hub, it is preferable to apply a separate catheter hub 70, which has the stepped structure, on which the locking pivot 50 to be described later is hung, as a component of the reuse-prevention catheter.

The inner needle 110 is a needle having a sharp tip and is inserted into the outer needle 71 by inserting and passing through the catheter hub 70. The cannula plug 10 is provided with the inner needle 110 and is configured to be separated from the catheter hub 70 while the inner needle 110 is withdrawn from the outer needle 71.

The cannula plug 10 may have a hollow cylindrical structure, but in this embodiment, it may have a rectangular structure with a hollow defined therein for the purpose of preventing rotation. The cannula plug 10 is provided with the inner needle 110 that is fixedly coupled therein. A separate inner needle housing may be provided inside the catheter plug 10 as a means that fixes the inner needle 110. In this case, the inner needle housing is disposed at a central portion of the inside of the cannula plug 10 so that a space is secured between an inner wall of the cannula plug 10 and an outer wall of the inner needle housing. The slide case 30, which will be described later, may be accommodated in the space.

The slide case 30 refers to a configuration that is coupled to the cannula plug 10 and accommodated inside the cannula plug 10 and also is discharged from the cannula plug 10 to cover the inner needle 110. The slide case 30 may be provided in a multistage structure including a first slide case 31 and a second slide case 33.

The slide case 30 may have a length dimension that is set according to a length of the inner needle 110. The slide case 30 may be provided in one stage with a long length. However, in this case, the length of the catheter plug 10 may also increase, resulting in increasing an overall length of a catheter product itself. In this embodiment, the slide case 30 is exemplified to have a two-stage structure constituted by the first slide case 31 and the second slide case 33.

The first slide case 31 may be accommodated inside the cannula plug 10 and may be provided with a latching part 310 that prevents the first slide case 31 from being separated when the first slide case 31 is discharged from the cannula plug 10. The second slide case 33 is accommodated inside the first slide case 31 and is provided with a latching part 330 that prevents the second slide case 33 from being separated when the second slide case 33 is discharged from the first slide case 31.

Each of the latching parts 310 and 330 of the first slide case 31 and the second slide case 33 refers to a structure that is provided at a rear end and is engaged with the end structure of the cannula plug 10 so as to be prevented from being separated. Thus, it is also acceptable to adopt the well-known interlocking structure. In this embodiment, the slide case 30 may be provided in a rectangular structure having a hollow therein. The slide case 30 may have a rectangular structure and be accommodated in the cannula plug 10 so as not to rotate relative to the cannula plug 10.

The block socket 40 refers to a component that is inserted into the rear end of the catheter hub 70, has a needle hole communicating with the catheter hub 70, and is coupled to the front end of the slide case 30. The block socket 40 refers to a socket in which a member that prevents reinsertion of the inner needle 110 is accommodated so as to prevent the inner needle 110 from being reused. The block socket 40 is fitted into and coupled to the catheter hub 70 and may be separated when predetermined force is applied thereto.

In the state of FIG. 2(a), the locking pivot 50 is accommodated within the block socket 40 and supported by the inner needle 110. Referring back to FIG. 3, when the inner needle 110 is withdrawn, a separate support means is removed from a lower end of the locking pivot 50 to secure an inner space of the block socket 40. FIG. 3 illustrates a state just before the cannula plug 10 is completely withdrawn, i.e., a moment when the block socket 40 and the catheter hub 70 are not separated from each other. However, at the moment when the catheter hub 70 is separated from the block socket 40, the locking pivot 50 rotates into the secured inner space of the block socket 40 as described above.

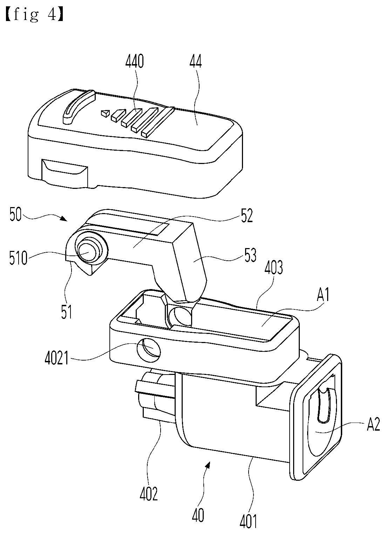

FIG. 4 is a view illustrating a block socket, the block socket 40, the locking pivot 50, and the socket cover 44 according to an embodiment of the present invention. Referring to FIG. 4, the block socket 40 may be divided into a first inner space A1, in which the front end on which a latching part 51 of the locking pivot 50 is disposed is accommodated, and a second inner space A2 in which the rear end on which the blocking part 53 of the locking pivot 50 is disposed is partially disposed and through which a needle hole communicates. The divided structure of the block socket 40 may be a structure in which spaces are defined vertically, and the first inner space A1 defined at an upper side is partitioned to be misaligned in a longitudinal direction so as to be displaced forward or backward relative to the second inner space A2.

The block socket 40 may include a hub coupling part 402, a socket housing 401, and a pivot housing 403. The hub coupling part 402, the socket housing 401, and the pivot housing 403 of the block socket 40 may be integrated by injection molding or machining.

The hub coupling part 402 refers to an area in which a needle hole is defined. The hub coupling part 402 is provided at a front end of the socket housing 401. The hub coupling part 402 may have an insertion and fixing structure on an outer circumference thereof and may be inserted in a direction of the rear end of the catheter hub 70 and then fixed and fixed inward. The hub coupling part 402 communicates with the second inner space A2 in a backward direction, and when the hub coupling part 402 is fitted into the catheter hub 70, the needle hole in a center thereof communicates with the catheter hub 70.

The socket housing 401 refers to a housing that defines the second inner space A2. The socket housing 401 may have a rectangular structure, and it is also acceptable to be provided in a circular structure. The socket housing 401 is coupled to the slide case 30 at a rear end thereof. The pivot housing 403 may extend upward from the socket housing 401.

The pivot housing 403 may be provided with a coupling hole 4021, to which the locking pivot 50 is rotatably coupled, in a sidewall thereof. The pivot housing 403 refers to a housing that defines the first inner space A1. The pivot housing 403 may allow the first inner space A1 to communicate in a ' 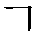 ' shape. Here, the "communication in the ' 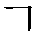 ' shape means a hollow structure in which the first inner space A1 is defined as a space in the longitudinal direction, and then, the second inner space A2 is opened in the downward direction. The pivot housing 403 is provided at an upper portion of the socket housing 401 and secures a ' 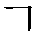 '-shaped path in the inner space of the block socket 40 to provide a minimum space in which the locking pivot 50 is rotatable.

The locking pivot 50 refers to a configuration that is provided with a latching part 51, which is accommodated inside the block socket 40 and engaged with the rear end of the catheter hub 70, at a side thereof and a blocking part 53 extending from the latching part 51 and bent in an inward direction of the block socket 40.

The locking pivot 50 is provided with a protrusion protruding from the latching part 51 so as to be engaged with the rear end of the catheter hub 70 in the first inner space A1 of the block socket 40. The locking pivot 50 may allow the latching part 51 to rotate in the outward direction due to external force through which the catheter plug 10 is separated from the catheter hub 70, thereby separating the catheter hub 70 from the block socket 40. As the locking pivot 50 rotates in the outward direction, the blocking part 53 may rotate inward into the second inner space A2 to block or shield the needle hole that communicates from the second inner space A2 to the catheter hub 70.

The locking pivot 50 may be provided with a hinge part 510 inserted into the coupling hole 4021 at the front end thereof to provide a fixed end at which the hooking part 51 is rotatable, and a blocking part 53 at the rear end thereof to provide a free end.

In the locking pivot 50, the hinge part 510 coupled to the block socket 40 may define the front end together with the latching part 51. The locking pivot 50 has an extension part 52 that transmits the rotational force of the latching part 51, and the blocking part 53 bent from the extension part 52 in the inward direction of the block socket 40 may define the rear end.

On the basis of the coupled state, in the lacking pivot 50, a length of the blocking part 53 that is the rear end may be less than a height of the second inner space A2 of the block socket 40. More preferably, when the locking pivot 50 is accommodated in the first inner space A1, a portion of the blocking part 53 is exposed to the second inner space A2. In this embodiment, the blocking part 53 that is partially exposed to the second inner space A2 may be provided to be less than half of the height of the second inner space A2. Due to the length design of the blocking part 53 of the locking pivot 50, a lower portion of the blocking part 53 may be supported from the inner needle 110 inserted into and passing through the second inner space A2. Although not shown in the drawings, in another embodiment, the blocking part 53 may be provided with a groove, on which the inner needle 110 is seated, at a lower end thereof, to enhance the supporting force of the inner needle 110.

The locking pivot 50 may be provided with the blocking part 53 bent in the inward direction of the block socket 40, and an angle defined between the extension part 52 and the blocking part 53 may be an obtuse angle including an angle of 90°. When the locking pivot 50 rotates, the blocking part 53 is in contact with a bottom surface of the second inner space A2. Once the blocking part 53 is in contact with the bottom surface of the second inner space A2, the blocking part 53 and the extension part 52 shield the needle hole path communicating with the second inner space A2. With this mechanism, once the inner needle 110 is withdrawn, the inner needle 110 may not penetrate the block socket 40 again due to the locking pivot 50, thereby preventing the reuse of the inner needle 110.

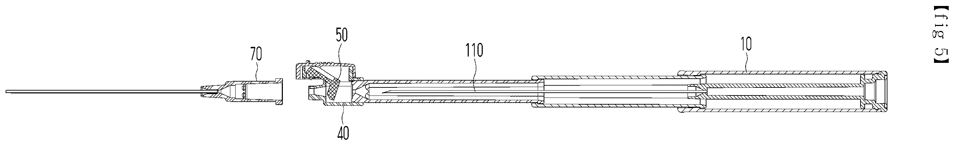

FIG. 5 is a view illustrating a state in which the cannula plug 10 of the reuse-prevention catheter is completely withdrawn to allow the locking pivot 50 to block a reinsertion path of the inner needle 110 according to an embodiment of the present invention.

The socket cover 44 may shield an upper portion of the block socket 40. An anti-slip protrusion 440 may be disposed on an upper end of the socket cover 44. The socket cover 44 is configured to be gripped by an index finger of a user. The user may use the index finger to push the anti-slip protrusion 440 of the socket cover 44 forward, and then use thumb and index fingers to grip the cannula plug 10 so as to pull out the inner needle 110.

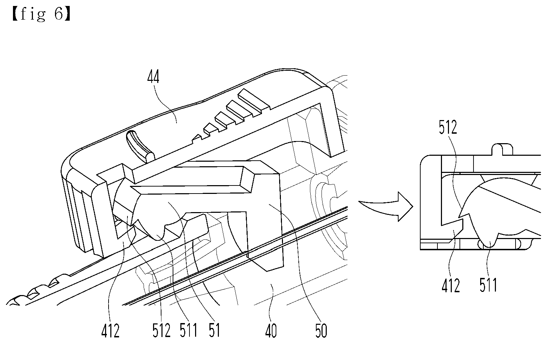

FIG. 6 is a view of the socket cover 44 according to an embodiment of the present invention. FIG. 6 is a side cross-sectional view of the socket cover 44 and the locking pivot 50 and illustrates a structure in which the socket cover 44 is coupled to the locking pivot 50.

Referring to FIG. 6, the socket cover 44 may be provided with a pivot stopper 412 protruding inward. The pivot stopper 412 may be provided in a locking structure protruding in the inward direction of the socket cover 44. In this embodiment, two protrusions may be provided on the locking pivot 51 of the locking pivot 50. The latching part 51 of the locking pivot 50 may include a hub latching part 511 and a socket latching part 512. The hub latching part 511 may have a protruding structure that is latched over the rear end of the catheter hub 50 as if being hung. When the catheter hub 50 moves forward, or the block socket 40 moves backward, the hub latching part 511 rotates in a clockwise direction with respect to FIG. 6. The socket latching part 512 may be provided as a protruding structure above the hub latching part 511 and may be coupled to the pivot stopper 412. When the hub latching part 511 rotates in the clockwise direction, the socket latching part 512 may be coupled to the pivot stopper 412, and thus, rotation of the hub latching part 511 in a counterclockwise direction may be blocked due to the inner protruding structure of the pivot stopper 412.

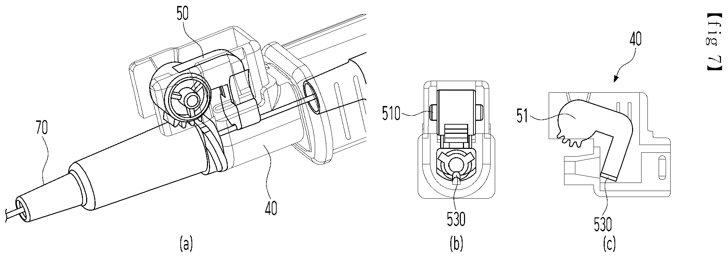

FIG. 7 is a view illustrating a block socket 40 and a locking pivot 50 according to another embodiment of the present invention. FIG. 7(a) is a transparent perspective view illustrating a block socket 40 and a locking pivot 50 according to another embodiment, FIG. 7(b) is a front view illustrating the block socket 40 and the locking pivot 50 according to another embodiment, and FIG. 7(c) is side view illustrating the block socket 40 and the locking pivot 50 according to another embodiment.

In an embodiment of FIG. 7, an embodiment in which the locking pivot 50 has a protrusion configured in a toothed shape on the latching part 51 is disclosed. Referring to FIG. 7, the locking pivot 50 may be constituted by a latching part 51, an extension part 52, and a blocking part 53. In this embodiment, the latching part 51 may be provided as a locking structure in which teeth are provided by several protrusions. In this case, a catheter hub 70 may have a plurality of protruding structures on a stepped portion of a rear end thereof so that the teeth of the latching part 51 are engaged with the protruding structures. The blocking part 53 may be bent perpendicular to the extension part, and a groove 530 on which a needle is seated may be defined in a lower end of the blocking part 53.

Although the embodiments described above have been used to illustrate the present invention and the technical idea of the present disclosure, the present invention and the technical idea of the present disclosure include various substitutions, modifications, and changes that can be made by those skilled in the art within the scope of the present invention and the technical idea of the present disclosure. Furthermore, it should be understood that such substitutions, modifications and variations are intended to be within the scope of the appended claims.

10: Cannula plug
110: Inner needle
70: Catheter hub
71: Outer needle
30: Slide case
31: First slide case
310, 330: Latching part
33: Second slide case
40: Block socket
44: Socket cover
412: Pivot stopper
401: Socket housing
402: Hub coupling part
4021: Coupling hole
403: Pivot housing
440: Anti-slip protrusion
50: Locking pivot
51: Latching part
510: Hinge
511: Hub latching part
512: Socket latching part
52: Extension part
53: Blocking part
90: Cap

### INDUSTRIAL APPLICABILITY

The catheter of the present invention ensures the single-use of the tool for injecting the drugs to the subject.

## Claims

1. A reuse-prevention catheter comprising:
a catheter hub provided with an outer needle;
an inner needle inserted into the outer needle by passing through the catheter hub;
a cannula plug provided with the inner needle and separated from the catheter hub while the inner needle is withdrawn from the outer needle;
a slide case coupled to the cannula plug, accommodated into the cannula plug, and discharged from the cannula plug to cover the inner needle;
a block socket fitted into a rear end of the catheter hub, having a needle hole that communicates with the catheter hub, and coupled to a front end of the slide case; and
a locking pivot accommodated inside the block socket, provided with a latching part engaged with a rear end of the catheter hub at one side thereof, and provided with a blocking part that extends from the latching part and is bent inward toward the block socket,
wherein, when the cannula plug is separated from the catheter hub, the locking pivot is pivoted so that the blocking part blocks the needle hole.

2. The reuse-prevention catheter of claim 1, wherein the slide case has a multistage structure comprising:
a first slide case which is accommodated inside the cannula plug and is provided with a latching part configured to prevent the first slide case from being separated when being discharged from the cannula plug; and
a second slide case which is accommodated inside the first slide case and is provided with a latching part configured to prevent the second slide case from being separated when being discharged from the first slide case.

3. The reuse-prevention catheter of claim 1, wherein the block socket is divided into a first inner space, in which a front end on which a latching part of the locking pivot is disposed is accommodated, and a second inner space, in which a rear end on which a blocking part of the locking pivot is partially disposed and through which the needle hole communicates.

4. The reuse-prevention catheter of claim 3, wherein the locking pivot is configured so that:
a protrusion protruding from the latching part is disposed to be engaged with a rear end of the catheter hub in the first space of the block socket;
the latching part rotates outward by external force, through which the cannula plug is separated from the catheter hub, to separate the catheter hub and the block socket from each other; and
as the latching part rotates outward, the blocking part rotates inward in the second space to block the needle hole.

5. The reuse-prevention catheter of claim 3, further comprising a socket cover configured to shield an upper portion of the block socket,
wherein the socket cover is provided with an anti-slip protrusion on a top surface thereof.

6. The reuse-prevention catheter of claim 3, wherein the block socket is configured so that:
a hub coupling part having the needle hole is provided on a front end of a socket housing that defines the second inner space; and
a pivot housing that allows the first inner space to communicate in a ' 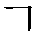 ' shape is provided on an upper portion of the socket housing.

7. The reuse-prevention catheter of claim 6, wherein the block socket has a coupling hole defined in a sidewall of the pivot housing so that the locking pivot is rotatably coupled, and
the locking pivot is provided with a hinge part inserted into the coupling hole on a front end thereof so that the latching part provides a rotatable fixed end, and the blocking part provides a free end.

8. The reuse-prevention catheter of claim 1, wherein the locking pivot has:
a front end defined together with the latching part by comprising a hinge part coupled to the block socket;
an extension part configured to transmit rotation force of the latching part; and
a rear end defined by the blocking part bent from the extension part in an inward direction of the block socket.

9. The reuse-prevention catheter of claim 8, wherein, in the locking pivot, the blocking part is bent in the inner direction of the block socket so that an angle between the extension part and the blocking part is in range of an obtuse angle comprising an angle of 90°.
